# EUROPÄISCHE PATENTSCHRIFT

(11) **EP 2 241 263 B1**
(45) Veröffentlichungstag und Bekanntmachung des Hinweises auf die Patenterteilung: **31.12.2014**
(21) Anmeldenummer: 10159743.3
(22) Anmeldetag: 13.04.2010
(51) Int. Cl.: A61B 17/04

(54) **Ankervorrichtung zum knotenfreien Fixieren von Gewebe an einem Knochen**
Anchor device for knot-free attachment of tissue to a bone
Dispositif d'ancrage destiné à la fixation sans noeud de tissu sur un os

(30) Priorität: 15.04.2009 DE 102009018136
(43) Veröffentlichungstag der Anmeldung: 20.10.2010
(73) Patentinhaber: Karl Storz GmbH & Co. KG, 78532 Tuttlingen (DE)
(72) Erfinder: Berberich, Sascha, 78532 Tuttlingen (DE); Ulmschneider, Rainer, 78532 Tuttlingen (DE); Gerber, Christian,Prof., 8126 Zumikon (CH)
(74) Vertreter: Witte, Weller & Partner Patentanwälte mbB

(56) Entgegenhaltungen:
- EP-A2- 2 160 983
- WO-A1-97/29693
- DE-A1-102006 010 116
- US-A1- 2005 245 932

## Beschreibung

Die Erfindung betrifft eine Ankervorrichtung zum knotenfreien Fixieren von Gewebe an einem Knochen mittels zumindest eines durch die Ankervorrichtung hindurchgefädelten Fadens, mit einem im distalen Endbereich eines Ankerkörpers angeordneten und durch diesen hindurchreichende Querbohrung zum Durchfädeln des zumindest einen Fadens quer durch den Ankerkörper hindurch, mit einer sich längs des Ankerkörpers erstreckenden und bis zur Querbohrung reichenden von einer Wand umgebenen Längsbohrung, und mit einem in der Längsbohrung aufnehmbaren axial bewegbaren Klemmelement zum Klemmen des durch den Ankerkörper hindurchgefädelten Fadens zwischen Klemmelement und Ankerkörper.

Eine derartige Ankervorrichtung ist aus der DE 10 2006 010 116 A1 bekannt.

Solche Ankervorrichtungen, auch Fadenanker genannt, werden im medizinischen Bereich dazu eingesetzt, um von einem Knochen abgelöstes Gewebe, meist Sehnen, wieder am Knochen zu fixieren.

Grundsätzlich wird dazu die Ankervorrichtung samt einem durch die Ankervorrichtung hindurchgefädelten Faden in den Knochen festsitzend eingetrieben. Der Faden wird einerseits mit dem abgelösten Gewebe verbunden, andererseits mit der Ankervorrichtung verklemmt, dadurch wird das losgelöste Gewebe am Knochen fixiert.

Bei der eingangs erwähnten DE 10 2006 010 116 A1 ist dazu ein Ankerkörper vorgesehen, welcher eine etwa zylindrische Form aufweist, an dessen Außenseite Vorsprünge vorgesehen sind, die ein Abziehen der Ankervorrichtung, nachdem sie in den Knochen eingebracht worden ist, verhindern. Diese Vorsprünge sind als widerhakenartige Elemente ausgebildet, so dass diese, nachdem der Ankerkörper in den Knochen eingeschlagen oder eingetrieben und der Faden verklemmt wurde, bei auftretender Zugbelastung eine Widerstandskraft gegen Abziehen des Ankerelements vom Knochen entwickeln.

Zunächst wird, meist unter Zuhilfenahme einer Nadel, der Faden durch das zu fixierende Gewebe hindurchgestochen. Dann werden die beiden freien Fadenenden durch die Querbohrung des Ankerkörpers hindurchgefädelt. Die Verbindung zwischen Ankerkörper und zu fixierendem Gewebe besteht in Form einer Fadenschleife. Der Ankerkörper kann schon jetzt samt Faden in den Knochen eingebracht werden und es wird anschließend an den freien Fadenenden gezogen, wodurch die vorstehende Schleife des Fadens, die mit dem Gewebe verbunden ist, an die zu fixierende Stelle herangezogen wird.

Zum Fixieren der Relativstellung zwischen Faden und damit verbundenem Gewebe und Ankerkörper wird nunmehr ein Klemmelement in eine senkrecht zur Querbohrung verlaufende Längsbohrung bewegt, wodurch der in der Querbohrung befindliche Abschnitt des Fadens in einer bestimmten Position am Ankerkörper fixiert, sprich geklemmt wird. Dadurch wird die Schleife, die das Gewebe hält, ebenfalls fixiert. Die überstehenden freien Enden können dann beispielsweise abgeschnitten werden.

Wird eine hoch beanspruchte Sehne, beispielsweise aus dem Schulterbereich oder dem Kniebereich, fixiert, so ist einleuchtend, dass erhebliche Zugkräfte von der Sehne auf den in den Knochen eingebrachten Zusammenbau aus Ankerkörper, Klemmelement und dazwischen geklemmten Faden einwirken.

Die von der Außenseite des Ankerkörpers vorstehenden Widerhaken nehmen ein zusätzliches Volumen ein. Beim Einschlagen des Ankerkörpers in den Knochen, im Vergleich zu einem vorsprunglosen Ankerkörper, ist die eingeschlagene Öffnung durch die Vorsprünge aufgeweitet. Die Vorsprünge erzeugen nach dem Einschlagen des Ankerelements nur eine geringe Druckkraft auf die Seitenwand der entstandenen Öffnung, wodurch aufgrund der Proportionalität zwischen Druckkraft und Widerstandskraft letztere ebenfalls nur schwach ansteigt.

Somit wird der von den Vorsprüngen erzeugte Zuwachs der Wandrauhigkeit des Ankerkörpers aufgrund der geringen Druckkraft beim reibenden Zusammenwirken mit der Seitenwand der Öffnung nur suboptimal ausgenutzt, mit dem Ergebnis, dass eine unzulängliche und unbefriedigende Verankerung erreicht wird.

Aus der EP 2 160 983 A2, die Stand der Technik nach Art. 54(3) EPÜ ist, ist eine Ankervorrichtung zum knotenfreien Fixieren von Gewebe an einem Knochen mittels zumindest eines durch die Ankervorrichtung hindurchgefädelten Fadens bekannt, mit einer im distalen Endbereich eines Ankerkörpers angeordneten und durch diesen hindurch reichenden Querbohrung zum Durchfädeln des zumindest einen Fadens quer durch den Ankerkörper hindurch, mit einer sich längs des Ankerkörpers erstreckenden und bis zur Querbohrung reichenden, von einer Wand umgebenen Längsbohrung, und mit einem in der Längsbohrung aufnehmbaren axial bewegbaren Klemmelement zum Klemmen des durch den Ankerkörper hindurchgefädelten Fadens zwischen Klemmelement und Ankerkörper. Es ist ein Spreizelement vorhanden, durch das zumindest ein Abschnitt der Wand des Ankerkörpers radial nach außen spreizbar ist

Weitere derartige Ankerelemente sind aus der US 2005/0245932 A1 und der WO 97/29693 A1 bekannt.

Es ist Aufgabe der vorliegenden Erfindung, eine Ankervorrichtung der eingangs genannten Art zu schaffen, die einfach aufgebaut ist und insbesondere eine wirksame und zuverlässige Verankerung der Ankervorrichtung sicherstellt.

Erfindungsgemäß wird die Aufgabe dadurch gelöst, dass ein Spreizelement vorhanden ist, durch das zumindest ein Abschnitt der Wand des Ankerkörpers radial nach außen spreizbar ist, wobei das Spreizelement am proximalen Endbereich des Klemmelementes angeordnet ist, und dass der spreizbare Abschnitt der Wand durch mehrere umfänglich verteilte, axial verlaufende Einschnitte in der Wand ausgebildet ist, und dass die Einschnitte sich vom proximalen Ende der Wand in Richtung auf die Querbohrung zu erstrecken.

Der Körper des Ankers kann in eine Kemlochbohrung im Knochen eingetrieben werden, deren lichter Innendurchmesser in etwa dem Außendurchmesser des Körpers entspricht. Durch die radial nach außen spreizbare Wand wird der Durchmesser in deren Spreizbereich größer als der Durchmesser der Kemlochbohrung. Der spreizbare Abschnitt der Wand wird radial in das Knochenmaterial eingetrieben und sperrt oder blockiert ein Abziehen des Ankers vom Knochen.

Der Körper des Ankers kann an seiner Außenseite glatt oder mit umlaufenden Rippen versehen sein. Entscheidend ist, dass ein Abschnitt der Wand, nach dem Spreizen, sich in das Knochenmaterial gegen Abziehen sperrend hineinbewegt hat.

Knochen sind so aufgebaut, dass der äußere Randbereich (Kortinalis) relativ hart ist und der weiter innen liegende Bereich (Spongiosa) relativ weich ist. Wird der Anker so weit eingetrieben, dass der gespreizte Bereich der Wand unter der harten Knochenrinde liegt, so blockiert dieser harte Knochenbereich zusätzlich ein Abziehen des Ankers. Der spreizbare Bereich der Wand kann weit in die Spongiosa eindringen.

Dabei ist das Spreizelement am Klemmelement ausgebildet.

Durch eine derartige Ausgestaltung wird die Anzahl der Bauteile reduziert der Fertigungsaufwand verringert und zugleich die Handhabung und Montage erleichtert wird. Dies eröffnet die Möglichkeit, mit ein und demselben Bauelement sowohl die Spreizung der Wand als auch das Klemmen des Fadens durchzuführen. Dies kann gleichzeitig erfolgen, so dass beide Vorgänge in einem Arbeitsvorgang und ggf. synchron durchgeführt werden können.

Ferner ist das Spreizelement am proximalen Endbereich des Klemmelements angeordnet.

Diese Maßnahme hat den Vorteil, dass das Spreizelement vom proximalen Ende des Ankerkörpers in Richtung distalem Ende eingeführt werden kann, und zunächst für seine eigentliche Aufgabe vorbereitet werden kann, nämlich den Faden zu klemmen. Erst wenn das Klemmelement schon nahezu vollständig in die Längsbohrung eingeführt ist, kommt das am proximalen Ende angeordnete Spreizelement zum Einsatz.

Durch das Vorsehen von Einschnitten können die spreizbaren Abschnitte genau definiert und ihre Spreizbarkeit erheblich erhöht werden. Dadurch wird ein definiertes Spreizen bei zugleich geringerer aufzuwendender Spreizkraft realisiert.

Dabei sind mehrere umfänglich verteilte, axial verlaufende Einschnitte in der Wand vorhanden.

Durch diese Maßnahme werden mehrere umfänglich verteilte Eingriffstellen mit der Seitenwand der Öffnung geschaffen, wodurch die Verankerung der Ankervorrichtung weiter verbessert wird.

Außerdem erstrecken sich die Einschnitte vom proximalen Ende der Wand in Richtung auf die Querbohrung zu.

Eine derartige Ausgestaltung des Ankerkörpers hat zum einen aus fertigungstechnischer Sicht den Vorteil, dass die Einschnitte mit sehr geringem Aufwand erzeugt werden können. Zum anderen weisen die spreizbaren Abschnitte am proximalen Ende eine freie Kante auf, welche sich nach dem Spreizen in den Knochen hineinfressen und bei Zugbelastung einen enormen Widerstand bieten kann.

In einer weiteren Ausgestaltung der Erfindung weist das Spreizelement einen radial erweiterten Abschnitt auf, dessen Außendurchmesser größer als der lichte Innendurchmesser der Längsbohrung ist.

Diese Maßnahme bietet eine einfache und effiziente Möglichkeit, auch bei kleinen Bauteilen von der axialen Bewegung des Spreizelements eine radial nach außen wirkende Spreizkraft auf den zu spreizenden Abschnitt bereitzustellen.

In einer weiteren Ausgestaltung der Erfindung geht der radial erweiterte Abschnitt über eine Schräge in einen durchmessergeringeren Abschnitt über.

Das Spreizelement kann die Form eines konusförmigen Keils aufweisen, wodurch die Keilwirkung ausgenutzt wird, um den zumindest einen spreizbaren Abschnitt radial nach außen zu spreizen. Die Spreizbewegung läuft dadurch harmonisch und nicht rückartig ab.

In einer weiteren Ausgestaltung der Erfindung ist ein Abschnitt der Wand, der mit dem radial erweiterten Abschnitt beim Spreizen in Eingriff kommt, entsprechend komplementär abgeschrägt.

Diese Maßnahme stellt eine funktionssichere und effektive Möglichkeit der Kraftübertragung vom Spreizelement auf den spreizbaren Abschnitt der Wand bereit.

In einer weiteren Ausgestaltung der Erfindung sind das Längenmaß des Klemmelementes und die Lage des Spreizelementes am Klemmelement so gewählt, dass das Spreizelement dann mit dem spreizbaren Abschnitt der Wand in spreizenden Eingriff bringbar ist, wenn ein distaler Endbereich des Klemmelementes in klemmendem Eingriff mit dem Faden bringbar ist.

Vorteilhaft an dieser Maßnahme ist, dass der Klemmvorgang und der Spreizvorgang optimal aufeinander abgestimmt sind, so dass beim Einbringen des Klemmelementes, an welchem das Spreizelement ausgebildet ist, Klemm- und Spreizvorgang zeitgleich und in einem Schritt vollzogen werden, wodurch die Handhabung erheblich erleichtert wird.

In einer weiteren Ausgestaltung der Erfindung weist der zumindest eine spreizbare Abschnitt der Wand ein sich umfänglich erstreckendes Scharnier auf.

Die Verschwenkbewegung verläuft definiert um das Scharnier.

Diese Maßnahme begünstigt die Spreizbarkeit der spreizbaren Abschnitte dahingehend weiter, dass die Materialbelastung des spreizbaren Abschnitts an der Verbindungsstelle zu der restlichen Wand minimiert und zudem die aufzuwendende Spreizkraft weiter reduziert wird. Somit wird ein mögliches Abbrechen der spreizbaren Abschnitte der Wand aufgrund von Materialbelastung bzw. ermüdung vermindert.

In einer weiteren Ausgestaltung der Erfindung erstreckt sich das Scharnier zwischen zwei axial verlaufenden Einschnitten.

Diese Maßnahme bietet den erheblichen Vorteil, dass ein durch die Einschnitte festgelegter Abschnitt der Wand durch den Scharnierverlauf eine definierte Spreizbewegung vollzieht.

In einer weiteren Ausgestaltung der Erfindung erstreckt sich das Scharnier um den gesamten Umfang.

Diese Maßnahme hat den Vorteil, dass der gesamte Umfang der Wand für die spreizbaren Abschnitte ausgenutzt werden kann, wodurch die Verankerung weiter optimiert wird.

In einer weiteren Ausgestaltung der Erfindung ist das Scharnier als Filmscharnier ausgebildet.

Diese Maßnahme hat in fertigungstechnischer Hinsicht den Vorteil, dass das Scharnier z.B. als Einstich ausgebildet bzw. bei Kunststoffen schon beim Spritzgießen erzeugt werden kann, wodurch die Herstellungskosten erheblich reduziert werden.

In einer weiteren Ausgestaltung der Erfindung sind das Klemmelement als Schraube, die in ein Innengewinde in der Längsbohrung eindrehbar ist, und das Spreizelement als erweiterter Schraubenkopf ausgebildet.

Eine derartige Ausgestaltung der Ankervorrichtung bietet eine simple, leicht handhabbare, kostengünstig herzustellende und effiziente Realisierung der Verankerung des Ankerkörpers und der Fixierung des Fadens. Dadurch können bei Ankergrößen im Bereich von einigen Millimetern erhebliche Zugkräfte aufgenommen werden. Es ist ja zu bedenken, dass durch den Abriss der zu fixierenden Sehne am Knochen schon eine Läsion existiert. Wenn es nun möglich ist, die Verankerung effektiv mit einem möglichst kleinen Ankerelement zu bewerkstelligen, ist das dem Heilungsprozess sehr zuträglich. Die kombinierte Klemm-/Spreiz-Vorrichtung erlaubt solche kleinen Bauweisen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:

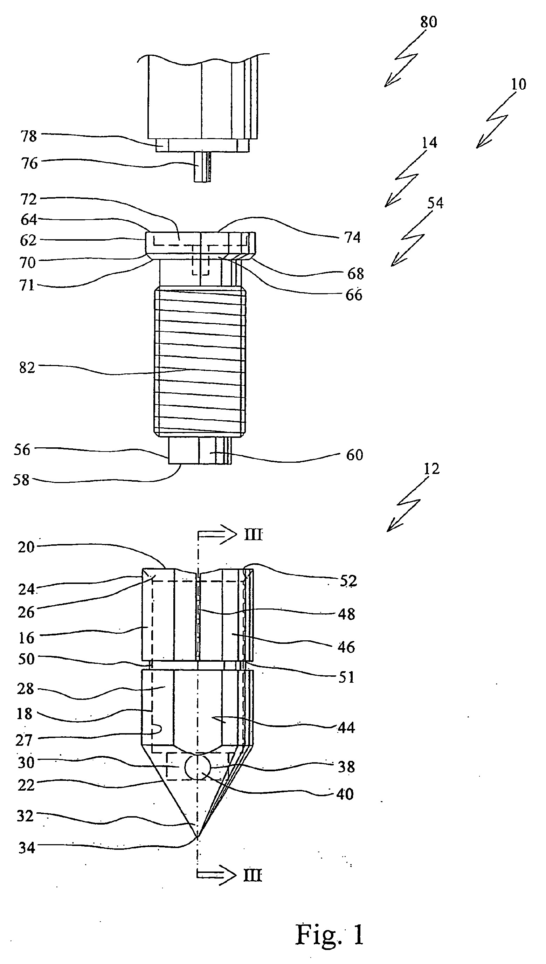

- Fig. 1: eine Explosionsdarstellung einer erfindungsgemäßen Ankervorrichtung und dessen Klemmelement, wobei am oberen Ende das Handhabungswerkzeug zum Setzen der Ankervorrichtung in den Knochen dargestellt ist,
- Fig. 2: eine perspektivische Darstellung des Klemmelements von Fig. 1,
- Fig. 3: eine Schnittdarstellung des Ankerkörpers von Fig. 1 längs der Linie III-III in Fig. 1,
- Fig. 4: eine perspektivische Darstellung des Ankerkörpers von Fig. 1 und Fig. 3,
- Fig. 5: eine Situation beim Setzen des Ankerelementes vor dem Zusammenfügen der Bauelemente,
- Fig. 6: eine Situation, bei der das Klemmelement in das Ankerelement eingedreht wird,
- Fig. 7: eine Situation, bei der das Klemmelement gerade auf den Faden trifft und das Spreizelement mit der zu spreizenden Wand in Eingriff tritt,
- Fig. 8: die Situation mit geklemmtem Faden und gespreiztem Wandabschnitt,
- Fig. 9: eine Detailansicht der Kontaktstelle zwischen der Ankervorrichtung und dem Knochen aus Fig. 7, wobei das am Klemmelement angeordnete Spreizelement gerade mit dem spreizbaren Abschnitt in Eingriff kommt,
- Fig. 10: den auf Fig. 9 folgenden Spreizvorgang, wobei ersichtlich ist, wie sich der spreizbare Abschnitt bei Zugbelastung in den Knochen hineinfrisst und ein Abziehen der Ankervorrichtung aus dem Knochen verhindert, und
- Fig. 11: einen Schnitt nach Setzen der Ankervorrichtung, Fixieren des Gewebes mittels des Fadens und Fixieren der Ankervorrichtung mittels des Spreizelementes in dem Knochen.

Eine in den Figuren dargestellte Ankervorrichtung zum knotenfreien Fixieren von Gewebe an einem Knochen mittels zumindest eines durch die Ankervorrichtung hindurchgefädelten Fadens ist in ihrer Gesamtheit mit der Bezugsziffer 10 bezeichnet.

Die Ankervorrichtung 10 weist einen Ankerkörper 12 und ein Klemmelement 14 auf.

Der Ankerkörper 12 weist eine langerstreckte grobzylindrische Form mit einer Wand 16 und einer Längsbohrung 18 auf. Die Längsbohrung 18 ist als Sacklochbohrung ausgebildet und erstreckt sich vom proximalen Ende 20 des Ankerkörpers 12 bis zum distalen Endbereich 22 des Ankerkörpers 12. Die Längsbohrung 18 weist einen am proximalen Endbereich 24 des Ankerkörpers 12 angeordneten ersten Längsbohrungsabschnitt 26, einen daran anschließenden bis zum distalen Endbereich 22 reichenden ein Innengewinde 27 aufweisenden zweiten Längsbohrungsabschnitt 28 sowie einen daran anschließenden am distalen Endbereich 22 angeordneten durchmessergeringeren dritten Längsbohrungsabschnitt 30 auf.

Der Ankerkörper 12 ist zudem an seinem distalen Endbereich 22 konisch ausgebildet und weist am distalen Ende 32 eine Ankerkörperspitze 34 auf. Die Ankerkörperspitze 34 dient dazu, den Ankerkörper 12 präzise und aufgrund der Spaltwirkung mit nur einem geringen Kraftaufwand in einen Knochen 36 (hier nicht dargestellt) anzusetzen und einzubringen.

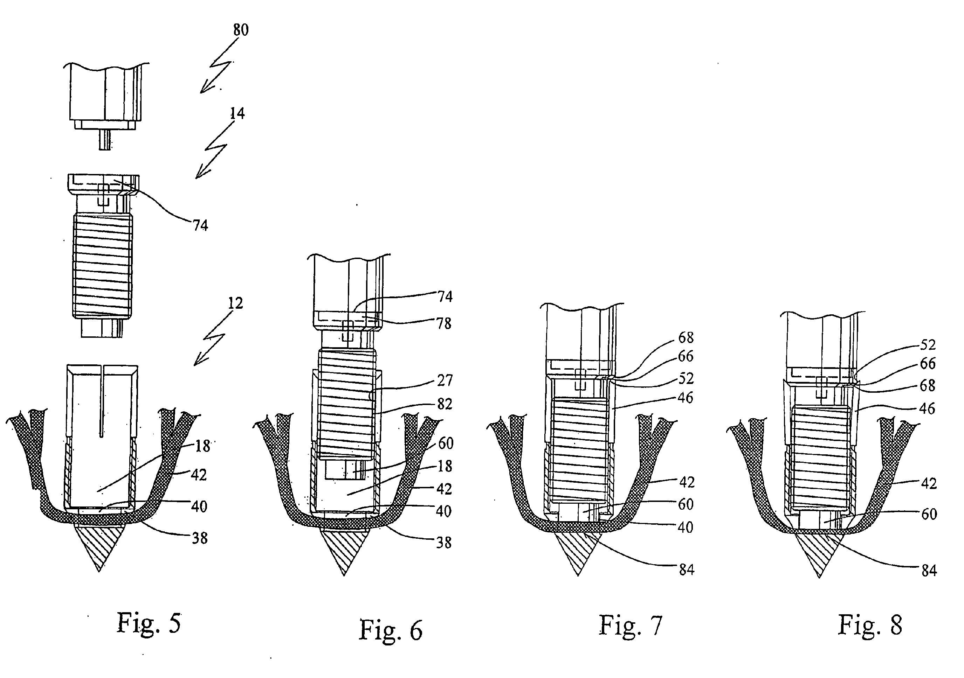

Ferner weist der Ankerkörper 12 an seinem distalen Endbereich 22 eine quer durch diesen hindurchreichende Querbohrung 38 auf, welche den dritten Längsbohrungsabschnitt 30 der Längsbohrung 18 durchdringt. Der gemeinsame Bereich zwischen der Querbohrung 38 und dem dritten Längsbohrungsabschnitt 30 wird dabei als Klemmkammer 40 definiert. Die Querbohrung 38 dient dazu einen Abschnitt eines Fadens 42 (hier nicht dargestellt) aufzunehmen, welcher durch das Klemmelement 14 in der Klemmkammer 40 fixiert wird, wie das nachfolgend bei der Beschreibung von Fig. 5 bis Fig. 8 detailliert erläutert wird.

Zwei sich gegenüberliegende seitlich angeordnete abgeflachte Seitenflächen 44 sind jeweils direkt oberhalb der Öffnungen der Querbohrung 38 angeordnet, und dienen dazu den Faden 42 nach Durchdringen der Querbohrung 38 jeweils beidseitig in Richtung proximalem Ende 20 des Ankerkörpers 12 aus dem Knochen 36 zu führen.

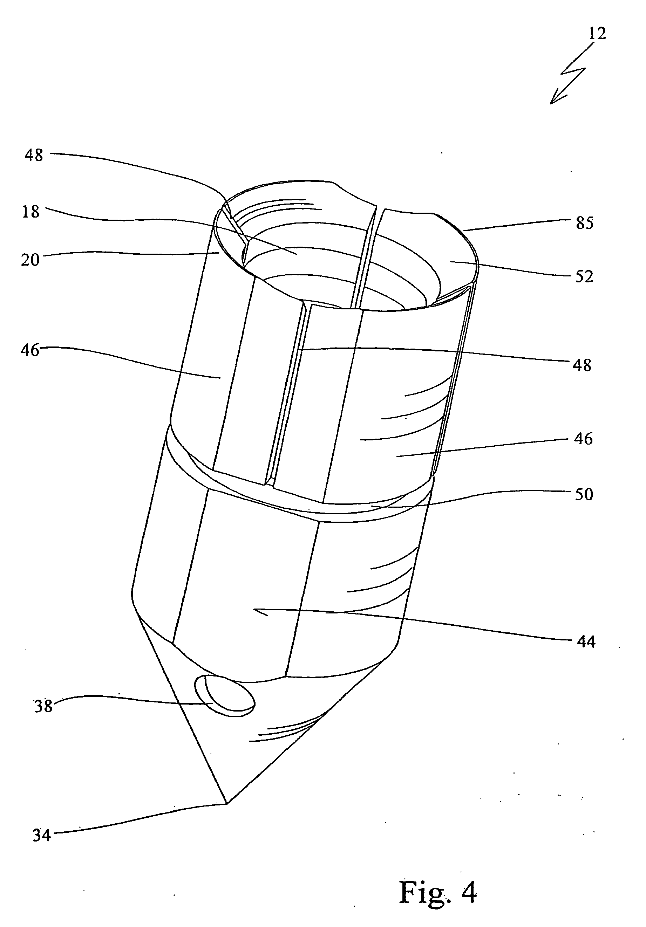

Der Ankerkörper 12 weist außerdem, wie aus Fig. 4 näher ersichtlich ist, vier am proximalen Endbereich 24 angeordnete spreizbare Abschnitte 46 auf, welche Bestandteile der Wand 16 sind. Die spreizbaren Abschnitte 46 sind gleichmäßig umfänglich an der Wand 16 des Ankerkörpers 12 verteilt und durch vier axiale Einschnitte 48, welche sich vom proximalen Ende 20 axial in Richtung auf die Querbohrung 38 zu erstrecken, ausgebildet.

Ein Scharnier 50 dient als elastische drehbare Anlenkung der spreizbaren Abschnitte 46 an die restliche Wand 16 des Ankerkörpers 12, so dass eine minimale Spreizkraft für das Spreizen der spreizbaren Abschnitte 46 aufgewendet werden muss. Das Scharnier 50 ist als Einstich 51 ausgebildet, welcher am Ende der Einschnitte 48 angeordnet ist und sich über den gesamten Umfang der Wand 16 erstreckt.

Die spreizbaren Abschnitte 46 sind am proximalen Ende 20 verjüngt ausgebildet und weisen im Bereich des ersten Längsbohrungsabschnittes 26 der Längsbohrung 18 eine nach distal abfallende Innenschräge 52 auf. Die Funktion dieser Innenschräge 52 wird in den nachfolgenden Figuren näher erläutert.

Das als Schraube 54 ausgebildete Klemmelement 14 weist einen an seinem distalen Endbereich 56 angeordneten und als zylindrischen Endschaft 58 ausgebildeten Klemmabschnitt 60 auf, welcher dazu ausgebildet ist, in die Klemmkammer 40 einzudringen, um den Faden 42 zu fixieren. Ferner weist das Klemmelement 14 ein an seinem proximalen Endbereich 62 angeordnetes als Schraubkopf 64 ausgebildetes Spreizelement 66 auf. Das Spreizelement 66 weist dabei eine zur Innenschräge 52 der spreizbaren Abschnitte 46 korrespondierende über den gesamten Umfang verlaufende Außenschräge 68 auf, welche von einem radial erweiterten Abschnitt 70 auf einen durchmessergeringeren Abschnitt 71 übergeht. Wie nachfolgend detaillierter dargelegt wird, wirkt die Außenschräge 68 bei einer axialen Bewegung des Spreizelementes 66 keilartig mit der Innenschräge 52 zusammen und spreizt dadurch die spreizenden Abschnitte 46 gleichzeitig radial nach außen.

Der Schraubenkopf 64 weist eine Nut 72 auf, welche als Werkzeugaufnahme 74 dient. Die Nut 72 ist derart ausgebildet, dass sie einen zylindrischen Zentrierzapfen 76 und einen Vorsprung 78 eines Eintreibers 80 aufnehmen kann, so dass formschlüssig ein Moment übertragen werden kann. Der Zentrierzapfen 76 dient dabei lediglich als Zentrierhilfe beim Eingriff des Eintreibers 80 in den Schraubenkopf 64 und leistet keinen Beitrag zur Momentenübertragung.

Das Klemmelement 14 weist zudem einen zwischen dem Schraubkopf 64 und dem zylindrischen Endschaft 58 angeordneten Außengewindeabschnitt 82 auf. Der Gewindeabschnitt 82 ist die Verbindungsstelle zwischen Klemmelement 14 und Ankerkörper 12 und überträgt somit beim Eindrehen in das Innengewinde 27 des Ankerkörpers 12 die Klemmkraft vom Klemmelement 14 auf den Faden 42 sowie die Spreizkraft vom Spreizelement 66 auf die spreizbaren Abschnitte 46.

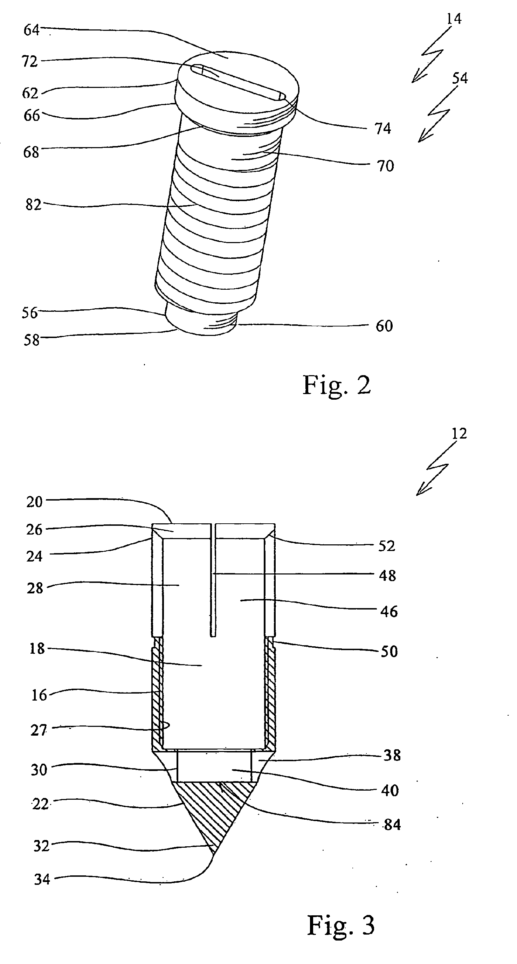

In Fig. 2 ist eine perspektivische Darstellung des als Schraube 54 ausgebildeten Klemmelements 14 dargestellt. Es ist ersichtlich, dass die Nut 72 eine teilweise schlitzförmige Form aufweist, so dass aufgrund der nicht rotationssymmetrischen Form ein Moment vom Eintreiber 80 über den Vorsprung 78 auf das Klemmelement 14 übertragen werden kann.

Der in Fig. 3 gezeigte Schnitt des Ankerkörpers 12 verdeutlicht nochmals die Form und Lage der Längsbohrung 18 sowie der Klemmkammer 40, wobei letztere, wie zuvor erläutert, als Schnittbereich zwischen der Querbohrung 38 und dem dritten Längsbohrungsabschnitt 30 der Längsbohrung 18 definiert ist. Zudem ist aus dieser Figur ersichtlich, dass die Klemmkammer 40 einen Klemmboden 84 aufweist, gegen den der Faden 42 mittels des Klemmabschnitts 60 des Klemmelements 14 gedrückt und somit fixiert wird.

Es sei angemerkt, dass der Klemmboden 84 eine beliebige zum Klemmabschnitt 60 des Klemmelements 14 korrespondierende Form aufweisen kann, wobei jedoch vorzugsweise der Klemmboden 84 flach und der Klemmabschnitt 60 ebenfalls flach mit einer abgerundeten Kante ausgebildet sind, so dass die Klemmkraft vom Klemmelement 14 über den Klemmabschnitt 60 flächig auf den Faden 42 übertragen wird. Durch die beschriebene Ausgestaltung wird die Gefahr der Beschädigung des Fadens 42 aufgrund punktueller Krafteinwirkung, wie es z.B. bei stark gekrümmten Formen der Fall ist, stark reduziert.

In Fig. 4 ist der Ankerkörper 12 perspektivisch dargestellt. Es ist ersichtlich, dass die vier spreizbaren Abschnitte 46 durch vier durch die Wand 16 reichende axial verlaufende Einschnitte 48 ausgebildet sind. Die Einschnitte 48 erstrecken sich dabei vom proximalen Ende 20 des Ankerkörpers 12 bis zum Einstich 50, welcher etwa auf Höhe der Mitte des Ankerkörpers 12 angeordnet ist.

Des Weiteren wird der Verlauf der Innenschräge 52 der spreizbaren Abschnitte 46 verdeutlicht. Dabei sei zudem angemerkt, dass die Innenschräge 52 eine stumpfe Kante 85 aufweist.

Um das Prinzip der vorliegenden Erfindung zu verdeutlichen, zeigen Fig. 5 bis Fig. 8 den Ablauf der Operationstechnik vom Setzen bis zum Fixieren der Ankervorrichtung 10 in den Knochen 36, wobei letzterer aus Darstellungsgründen nicht dargestellt ist.

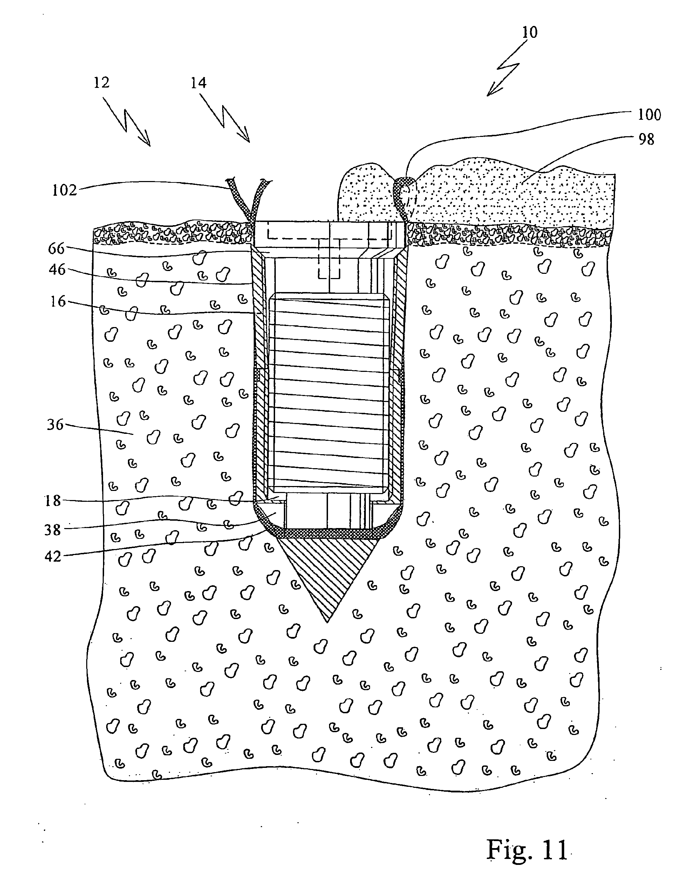

Fig. 5 zeigt eine Explosionsdarstellung mit dem Ankerkörper 12, dem oberhalb der Längsbohrung 18 angeordneten Klemmelement 14 und dem oberhalb der Werkzeugaufnahme 74 angeordneten Eintreiber 80. Der Faden 42 ist dabei durch die Querbohrung 38 durchgefädelt und durchdringt somit die Klemmkammer 40. Wie nachfolgend aus der Beschreibung von Fig. 11 näher ersichtlich wird, durchquert der Faden 42 die Querbohrung 38 zweimal, d.h. jeweils einmal in entgegengesetzter Richtung, so dass auf einer Seite eine Schleife entsteht, die zuvor mit dem zu fixierenden Gewebe verbunden wurde, beispielsweise durch einen Nadeleinstich. Da die beiden Fadenabschnitte im Ankerkörper hintereinander liegen, ist in der Figurenfolge von Fig. 5 - 8 nur ein Fadenabschnitt zu erkennen.

Fig. 6 zeigt eine nachfolgende Momentaufnahme, bei der der Vorsprung 78 des Eintreibers 80 in Eingriff mit der Werkzeugaufnahme 74 des Klemmelements 14 steht und das Klemmelement 14 über den Gewindeabschnitt 82 in die das Innengewinde 27 aufweisende Längsbohrung 18 eingedreht wird. Dabei nähert sich der Klemmabschnitt 60 der Klemmkammer 40 und somit dem zu klemmenden Faden 42.

Die in Fig. 7 gezeigte Momentaufnahme stellt den Zeitpunkt dar, an dem der Klemmabschnitt 60 in klemmenden Eingriff mit dem Faden 42 und das Spreizelement 66 in spreizenden Eingriff mit den spreizbaren Abschnitten 46 kommt, d.h. den Beginn des Klemm- und Spreizvorgangs.

Es ist ersichtlich, dass zu dem Zeitpunkt, an dem der Klemmabschnitt 60 den Faden 42 erreicht und gegen den Klemmboden 84 drückt, zeitgleich die Innenschräge 52 des Spreizelementes 66 in die Außenschräge 68 der spreizbaren Abschnitte 46 eingreift.

Der letzte Schritt, d.h. die Fixierung des Fadens 42 und die Verankerung der Ankervorrichtung ist in Fig. 8 gezeigt. Während durch die axiale Bewegung des Klemmelements 14 der Klemmabschnitt 60 den Faden 42 gegen den Klemmboden 84 presst und somit fixiert, wirkt das Spreizelement 66 spreizend mit den spreizbaren Abschnitten 46 zusammen. Wie bereits zuvor erläutert, wird durch das Eingreifen der Außenschräge 68 des Spreizelementes 66 in die korrespondierende Innenschräge 52 der spreizbaren Abschnitte 46 eine Keilwirkung erzeugt, welche die spreizbaren Abschnitte 46 gleichzeitig radial nach außen spreizt, wodurch die Ankervorrichtung 10 im Knochen 36 verankert wird.

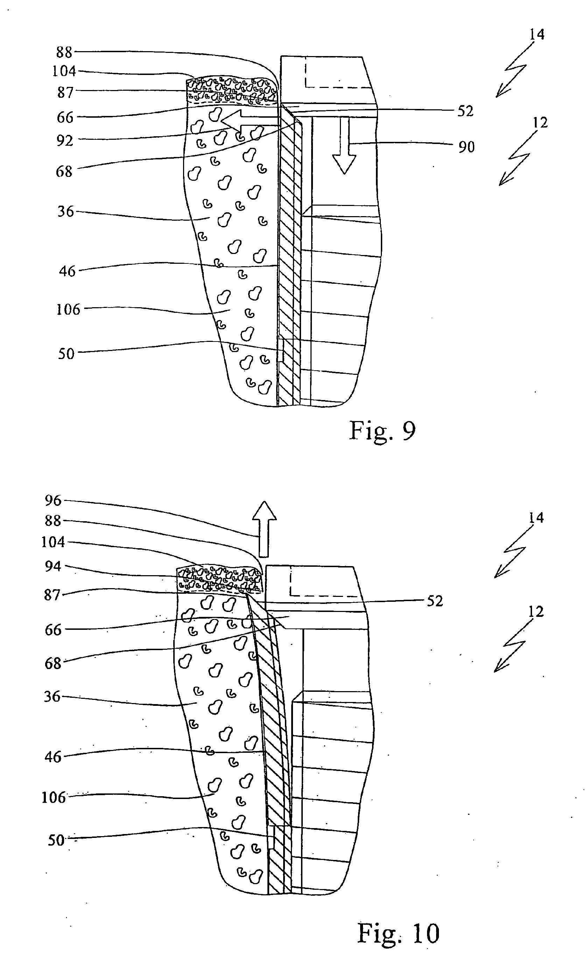

Fig. 9 und Fig. 10 sind Detailansichten von Fig. 7 bzw. Fig. 8 und verdeutlichen den spreizenden Eingriff des Spreizelements 66 mit einem spreizbaren Abschnitt 46 sowie das Hineinfressen des spreizbaren Abschnitts 46 in eine Seitenwand 87 einer Öffnung 88 des Knochens 36.

In Fig. 9 ist dargestellt, dass der Ankerkörper 12 so weit in eine zuvor bewerkstelligte Kernlochbohrung eingetrieben wurde, dass das proximale Ende unter der relativ harten Knochenrinde 104 zum Liegen kommt. Weiter innen im Knochen 36 ist das Knochenmaterial 106 wesentlich weicher (spongiös).

In Fig. 9 ist eine Momentaufnahme zu Beginn des Spreizvorgangs dargestellt. Die axiale Bewegungsrichtung des Klemmelements 14 wird dabei durch den Pfeil 90 veranschaulicht. Die aufgrund der Keilwirkung erzeugte Spreiz- oder Radialkraft erzeugt dabei mit fortschreitendem Eindringen des Spreizelementes 66 in die Längsbohrung 18 eine Spreizbewegung des spreizbaren Abschnitts 46, dessen Bewegungsrichtung durch einen Pfeil 92 veranschaulicht ist.

Aus Fig. 10 wird deutlich, wie sich die Ankervorrichtung 10 durch den gespreizten Abschnitt 46 in diesem Bereich aufweitet und der spreizbare Abschnitt 46, insbesondere im Bereich des proximalen Endes 20 in die Seitenwand 87 der Öffnung 88 eindringt und sich in das relativ weiche Material 106 des Knochens 36 hineinfrisst und dadurch die Ankervorrichtung 10 in der Öffnung 88 des Knochens 36 fixiert. Aus Fig. 10 ist zu entnehmen, dass der proximale Bereich der gespreizten Wand 46 nunmehr unter der harten Knochenrinde 104 zum Liegen kommt. Dadurch blockiert die Knochenrinde 104 zusätzlich gegen ein Abziehen des Ankerkörpers 12. Das Aufweiten des Ankerkörpers 12 im Bereich des spreizbaren Abschnitts 46 führt demzufolge zu einem Zuwachs der Druckkraft auf die Seitenwand 87 der Öffnung 88, was wiederum einen starken Anstieg der Widerstandskraft zur Folge hat.

Wie aus Fig. 10 ferner ersichtlich, weist die Innenschräge 52 des spreizbaren Abschnitts 46 eine weitere Funktion auf. Während die Innenschräge 52 zum einen mit der Außenschräge 68 des Spreizelements 66 zusammenwirkt, verstärkt sie zum anderen durch ihre Form und proximale Lage das Hineinfressen in den Knochen 36, wodurch die Widerstandscharakteristik der Ankervorrichtung 10 in der Öffnung 88 deutlich zunimmt. Dies wird dadurch erreicht, dass sich der spreizbare Abschnitt 46 durch die zugespitzte Form am proximalen Ende 20 leichter in den Knochen 36 hineinfressen und sich dadurch an einen proximalen Wandabschnitt 94 des Knochens 36 stützen kann. Demzufolge wird im Falle einer durch einen Pfeil 96 veranschaulichten Zugbelastung eine zusätzliche Blockierung und somit Widerstandskraft durch die gespreizten Abschnitte 46 bereitgestellt.

Fig. 11 zeigt schließlich einen Schnitt nach dem Setzen der Ankervorrichtung 10, dem Fixieren eines Gewebes 98, beispielsweise einer Sehne, mittels des Fadens 42, dem Fixieren des Fadens 42 mittels des Klemmelementes 14 und der Verankerung der Ankervorrichtung 10 mittels des Spreizelementes 66 in der Öffnung 88 des Knochens 36.

Dabei ist ersichtlich, dass das Gewebe 98 durch eine vom Faden 42 gebildete Schleife 100, welche das Gewebe 98 teilweise durchdringt, gehalten wird. Zwei Fadenenden 102 sollen verdeutlichen, dass der überschüssige Fadenabschnitt des Fadens 42 nach Fixieren des Fadens 42 abgeschnitten wird.

## Patentansprüche

1. Ankervorrichtung zum knotenfreien Fixieren von Gewebe (98) an einem Knochen (36) mittels zumindest eines durch die Ankervorrichtung hindurchgefädelten Fadens (42), mit einer im distalen Endbereich (22) eines Ankerkörpers (12) angeordneten und durch diesen hindurchreichenden Querbohrung (38) zum Durchfädeln des zumindest einen Fadens (42) quer durch den Ankerkörper (12) hindurch, mit einer sich längs des Ankerkörpers (12) erstreckenden und bis zur Querbohrung (38) reichenden von einer Wand (16) umgebenen Längsbohrung (18), und mit einem in der Längsbohrung (18) aufnehmbaren axial bewegbaren Klemmelement (14) zum Klemmen des durch den Ankerkörper (12) hindurchgefädelten Fadens (42) zwischen Klemmelement (14) und Ankerkörper (12), wobei ein Spreizelement (66) vorhanden ist, durch das zumindest ein Abschnitt (46) der Wand (16) des Ankerkörpers (12) radial nach außen spreizbar ist, dass das Spreizelement (66) am proximalen Endbereich (62) des Klemmelements (14) angeordnet ist und dass der spreizbare Abschnitt (46) der Wand (16) durch mehrere umfänglich verteilte, axial verlaufende Einschnitte (48) in der Wand (16) ausgebildet ist, und dass die Einschnitte (48) durch die Wand (16) hindurchgehen und sich vom proximalen Ende (20) der Wand (16) in Richtung auf die Querbohrung (38) zu erstrecken.

2. Ankervorrichtung nach Anspruch 1, **dadurch gekennzeichnet, dass** das Spreizelement (66) einen sich radial erweiterten Abschnitt (70) aufweist, dessen Außendurchmesser größer als der lichte Innendurchmesser der Längsbohrung (18) ist.

3. Ankervorrichtung nach Anspruch 2, **dadurch gekennzeichnet, dass** der radial erweiterte Abschnitt (70) über eine Schräge (68) in einen durchmessergeringeren Abschnitt (71) übergeht.

4. Ankervorrichtung nach Anspruch 3, **dadurch gekennzeichnet, dass** ein Abschnitt (52) der Wand (16), der mit dem radial erweiterten Abschnitt (70) beim Spreizen in Eingriff kommt entsprechend komplementär abgeschrägt ist.

5. Ankervorrichtung nach einem der Ansprüche 1 bis 4, **dadurch gekennzeichnet, dass** das Längenmaß des Klemmelementes (14) und die Lage des Spreizelementes (66) am Klemmelement (14) so gewählt sind, dass das Spreizelement (66) dann mit dem spreizbaren Abschnitt (46) der Wand (16) in spreizendem Eingriff bringbar ist, wenn ein distaler Endbereich (56) des Klemmelementes (14) in klemmendem Eingriff mit dem Faden (42) bringbar ist.

6. Ankervorrichtung nach einem der Ansprüche 1 bis 5, **dadurch gekennzeichnet, dass** der zumindest eine spreizbare Abschnitt (46) der Wand (16) ein umfänglich erstreckendes Scharnier (50) aufweist.

7. Ankervorrichtung nach Anspruch 6, **dadurch gekennzeichnet**, sich dass das Scharnier (50) zwischen zwei axial verlaufenden Einschnitten (48) erstreckt.

8. Ankervorrichtung nach Anspruch 6 oder 7, **dadurch gekennzeichnet, dass** sich das Scharnier (50) um den gesamten Umfang der Wand (16) erstreckt.

9. Ankervorrichtung nach einem der Ansprüche 6 bis 8, **dadurch gekennzeichnet, dass** das Scharnier (50) als Filmscharnier ausgebildet ist.

10. Ankervorrichtung nach einem der Ansprüche 1 bis 9, **dadurch gekennzeichnet, dass** das Klemmelement (14) als Schraube (54) ausgebildet ist, die in ein Innengewinde (27) in der Längsbohrung (18) eindrehbar ist, und dass das Spreizelement (66) als erweiterter Schraubenkopf (64) ausgebildet ist.

## Claims

1. Anchor device for not-free attachment of tissue (98) to a bone (36) by means of at least one suture (42) threaded through the anchor device, with a transverse bore (38) arranged in the distal end section (22) of an anchor body (12) and reaching through the latter for threading the at least one suture (42) transversely through the anchor body (12), with a longitudinal bore (18) surrounded by a wall (16) and extending along the anchor body (12) and extending up to the transverse bore (38), and with an axially displaceable clamping element (14), which can be held in the longitudinal bore (18) for clamping the suture (42) threaded through the anchor body (12) between the clamping element (14) and the anchor body (12), wherein there is a spreading element (66), by means of which at least one section (46) of the wall (16) of the anchor body (12) can be spreaded radially outward, wherein the spreading element (66) is arranged on the proximal end section (62) of the clamping element (14), and wherein the spreadable section (46) of the wall (16) is provided by a plurality of incisions (48) in the wall (16), which incisions are distributed over the circumference and run in the axial direction, and wherein the incisions (48) entirely pass the wall (16) and extend from the proximal end (20) of the wall (16) in the direction toward the transverse bore (38).

2. Anchor device of claim 1, **characterized in that** the spreading element (66) has a radially extended section (70), the external diameter of which being greater than the clear internal diameter of the longitudinal bore (18).

3. Anchor device of claim 2, **characterized in that** the radially extending section (70) merges into a section (71) with a smaller diameter via a bevel (68).

4. Anchor device of claim 3, **characterized in that** a section (52) of the wall (16), which engages with the radially extended section (70) when spreaded, has a corresponding complementary bevel.

5. Anchor device of anyone of claims 1 to 4, **characterized in that** the longitudinal measure of the clamping element (14) and the position of the spreading element (66) on the clamping element (14) are selected such that the spreading element (66) then can be brought into spreading engagement with the spreadable section (46) of the wall when a distal end section (22) of the clamping element (14) is brought into clamping engagement with the suture (42).

6. Anchor device of anyone of claims 1 to 5, **characterized in that** the at least one spreadable section (46) of the wall (16) has a hinge (50) extending around the circumference.

7. Anchor device of claim 6, **characterized in that** the hinge (50) extends between two incisions (48) running in the axial direction.

8. Anchor device of claims 6 or 7, **characterized in that** the hinge (50) extends over the entire circumference of the wall (16).

9. Anchor device of anyone of claims 6 to 8, **characterized in that** the hinge (50) is designed as a firm hinge.

10. Anchor device of anyone of claims 1 to 9, **characterized in that** the clamping element (14) is designed as a screw (54) which can be screwed into an inner thread (27) in the longitudinal bore (18), and **in that** the spreading element (66) is designed as an extended screw head (64).

## Revendications

1. Dispositif d'ancrage dévolu à l'assujettissement d'un tissu (98) à un os (36) avec absence de noeuds, à l'aide d'au moins un fil (42) enfilé à travers ledit dispositif d'ancrage, comprenant un alésage transversal (38) pratiqué dans la région extrême distale (22) d'un corps d'ancrage (12), traversant cette dernière de part en part et destiné à l'enfilement, transversalement à travers ledit corps d'ancrage (12), du fil (42) à présence minimale ; un alésage longitudinal (18) qui s'étend le long dudit corps d'ancrage (12), gagne ledit alésage transversal (38) et est entouré par une paroi (16) ; et un élément de coincement (14) mobile axialement, pouvant être logé dans ledit alésage longitudinal (18) et conçu pour coincer, entre ledit élément de coincement (14) et ledit corps d'ancrage (12), ledit fil (42) enfilé à travers ledit corps d'ancrage (12), sachant qu'il est prévu un élément d'écartement (66) par l'intermédiaire duquel au moins un segment (46) de la paroi (16) du corps d'ancrage (12) peut être déployé vers l'extérieur dans le sens radial, sachant que ledit élément d'écartement (66) est situé dans la région extrême proximale (62) de l'élément de coincement (14), sachant que ledit segment déployable (46) de la paroi (16) est constitué de plusieurs entailles (48) qui sont réparties sur le pourtour, s'étendent axialement et sont ménagées dans ladite paroi (16), et sachant que lesdites entailles (48) traversent intégralement la paroi (16) et s'étendent à partir de l'extrémité proximale (20) de ladite paroi (16), en direction de l'alésage transversal (38).

2. Dispositif d'ancrage selon la revendication 1, **caractérisé par le fait que** l'élément d'écartement (66) présente un tronçon (70) élargi dans le sens radial, dont le diamètre extérieur est plus grand que le diamètre intérieur de l'alésage longitudinal (18).

3. Dispositif d'ancrage selon la revendication 2, **caractérisé par le fait que** le tronçon (70) à élargrissement radial fusionne, par un biseau (68), dans un tronçon (71) de diamètre moindre.

4. Dispositif d'ancrage selon la revendication 3, **caractérisé par le fait qu'**un segment (52) de la paroi (16) venant en prise avec le tronçon (70) à élargissement radial, lors du déploiement, comporte un biseau de configuration adéquatement complémentaire.

5. Dispositif d'ancrage selon l'une des revendications 1 à 4, **caractérisé par le fait que** la cote longitudinale de l'élément de coincement (14), et la position de l'élément d'écartement (66) sur ledit élément de coincement (14), sont choisies de façon telle que ledit élément d'écartement (66) puisse être mis en prise avec le segment déployable (46) de la paroi (16), avec effet de déploiement, lorsqu'une région extrême distale (56) dudit élément de coincement (14) peut être mise en prise avec le fil (42), avec effet de coincement.

6. Dispositif d'ancrage selon l'une des revendications 1 à 5, **caractérisé par le fait que** le segment déployable (46), à présence minimale sur la paroi (16), est pourvu d'une charnière (50) s'étendant sur le pourtour.

7. Dispositif d'ancrage selon la revendication 6, **caractérisé par le fait que** la charnière (50) s'étend entre deux entailles (48) à tracés axiaux.

8. Dispositif d'ancrage selon la revendication 6 ou 7, **caractérisé par le fait que** la charnière (50) s'étend sur l'intégralité du pourtour de la paroi (16).

9. Dispositif d'ancrage selon l'une des revendications 6 à 8, **caractérisé par le fait que** la charnière (50) est réalisée sous la forme d'une charnière pelliculaire.

10. Dispositif d'ancrage selon l'une des revendications 1 à 9, **caractérisé par le fait que** l'élément de coincement (14) est réalisé sous la forme d'un vis (54) pouvant être vissée dans un filetage intérieur (27) façonné dans l'alésage longitudinal (18) ; et **par le fait que** l'élément d'écartement (66) est réalisé sous la forme d'une tête de vis élargie (64).
